# EUROPEAN PATENT APPLICATION

(11) **EP 4 230 210 A1**
(43) Date of publication of application: **23.08.2023**
(21) Application number: 21880135.5
(22) Date of filing: 13.10.2021
(51) Int. Cl.: A61K 31/5513, A61K 9/06, A61K 9/08, A61K 47/12, A61K 47/18, A61P 27/02

(54) **STABLE PHARMACEUTICAL COMPOSITION**

(30) Priority: 14.10.2020 JP 2020173195
(71) Applicant: Santen Pharmaceutical Co., Ltd., Kita-ku Osaka-shi Osaka 530-8552 (JP)
(72) Inventor: ENDO, Yoko, Ikoma-shi, Nara 630-0101 (JP); MASAKI, Kenji, Ikoma-shi, Nara 630-0101 (JP)
(74) Representative: Müller-Boré & Partner Patentanwälte PartG mbB
(86) International application number: PCT/JP2021/037841
(87) International publication number: WO 2022/080397

(57) **Abstract**

The present invention provides a stable pharmaceutical composition comprising (+)-11-[2-[2-[(diethylamino)methyl]-1-piperidinyl]acetyl]-5,11-dihydro-6H-pyrido[2,3-b][1,4]benzodiazepin-6-one or a salt thereof and a method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof.

## Description

### TECHNICAL FIELD

The present invention relates to a stable pharmaceutical composition comprising (+)-11-[2-[2-[(diethylamino)methyl]-1-piperidinyl]acetyl]-5,11-dihydro-6H-pyrido[2,3-b] [1,4]benzodiazepin-6-one (hereinafter also referred to as "AFDX0250") or a salt thereof (hereinafter also referred to as "the compound of the present invention") and a method of stabilizing AFDX0250 or a salt thereof in the pharmaceutical composition.

### BACKGROUND ART

When a compound useful as an active pharmaceutical ingredient is developed as a medicament, it has been required to satisfy various requirements such as the stability of the compound in a pharmaceutical composition, the stability of a pharmaceutical composition itself, ensuring the preservative effect in the medicament besides the effectiveness and safety of the medicament. In order to produce the stable effect of medicaments, it is extremely important to ensure the stability of the compound in a pharmaceutical composition among them.

The compound useful as an active pharmaceutical ingredient may be unstable in a pharmaceutical composition's state such as aqueous solution even when the compound itself in a solid state is stable. In such case, this may cause problems such as the production of degradation products and the increase in the amount of the produced degradation products. For example, when a compound is reacted with water in an ingredient such as pharmaceutical additive and degraded, the compound may become unstable in a pharmaceutical composition. Also, the stability of a pharmaceutical composition may be affected by various physical factors such as pH, heat, humidity, light. Hence, it is also important that a pharmaceutical composition is stable against such various factors.

On the other hand, the stability of a compound in a pharmaceutical composition is often revealed as problems only after the pharmaceutical composition is actually prepared. Hence, no general method of stabilizing a compound in a pharmaceutical composition is established and it is usual to find stabilization methods suitable for each compound.

Patent Document 1 discloses that 11-[2-[2-[(diethylamino)methyl]-1-piperidinyl]acetyl]-5,11-dihydro-6H-pyrido[2,3-b] [1,4]benzodiazepin-6-one (hereinafter also referred to as "AFDX0116") is useful for the treatment of glaucoma and/or ocular hypertension and that intraocular pressure is decreased by intracameral injection. Also, it is disclosed that one example of compounds for decreasing intraocular pressure includes AFDX0250 which is dextrorotatory (+)-enantiomer of AFDX0116.

However, the stability of AFDX0250 or a salt thereof in a pharmaceutical composition has not been reported. Hence, there is no report that when AFDX0250 or a salt thereof is prepared as a pharmaceutical composition, the compound becomes unstable in the pharmaceutical composition. Also, a method of stabilizing the compound has not been reported.

### PRIOR ART DOCUMENTS

### PATENT DOCUMENTS

Patent Document 1: WO 93/18772

### SUMMARY OF INVENTION

### (PROBLEM TO BE SOLVED BY THE INVENTION)

The present inventors have found at the stage of developing a pharmaceutical composition comprising AFDX0250 or a salt thereof that when the compound of the present invention is dissolved and prepared in the form of an aqueous composition, the compound is susceptible to hydrolysis and 5,11-dihydro-6H-pirido[2,3-b] [1,4]benzodiazepin-6-one, which is a hydrolysate of AFDX0250, is easily precipitated. In addition, the present inventors have found that the compound of the present invention is easily degraded under the light irradiation.

An object of the present invention is to provide a pharmaceutical composition comprising the compound of the present invention that inhibits the hydrolysis of the compound and thus inhibits the production of a hydrolysate thereof, the pharmaceutical composition that is stable to light, and a method of stabilizing the compound of the present invention.

### (MEANS FOR SOLVING THE PROBLEMS)

The present inventors have intensively studied to reach the aforementioned object, and have found that the adjustment of the pH of a pharmaceutical composition comprising the compound of the present invention to 6.5 or less can inhibit the hydrolysis of the compound of the present invention and thus inhibit the production of a hydrolysate thereof and that the addition of one or more buffering agents selected from the group consisting of citric acid, acetic acid, malic acid, an amine, an amino acid and a salt thereof improves the light stability of the compound of the present invention. Based upon the new findings, the present invention has been completed.

Specifically, the present invention provides the following embodiments.
(1) A pharmaceutical composition with a pH of 6.5 or less comprising (+)-11-[2-[2-[(diethylamino)methyl]-1-piperidinyl]acetyl]-5,11-dihydro-6H-pyrido[2,3-b] [1,4]benzodiazepin-6-one or a salt thereof.
(2) The pharmaceutical composition according to the above item (1), wherein the pH is 4 to 6.5.
(3) A pharmaceutical composition comprising (+)-11-[2-[2-[(diethylamino)methyl]-1-piperidinyl]acetyl]-5,11-dihydro-6H-pyrido[2,3-b] [1,4]benzodiazepin-6-one or a salt thereof and one or more buffering agents selected from the group consisting of citric acid, acetic acid, malic acid, an amine, an amino acid and a salt thereof.
(4) The pharmaceutical composition according to the above item (1) or (2), which comprises one or more buffering agents selected from the group consisting of citric acid, acetic acid, malic acid, an amine, an amino acid and a salt thereof.
(5) The pharmaceutical composition according to the above item (3) or (4) comprising citric acid or a salt thereof, wherein the pharmaceutical composition further comprises any of acetic acid or a salt thereof, malic acid or a salt thereof, an amine or a salt thereof, or an amino acid or a salt thereof as a buffering agent.
(6) The pharmaceutical composition according to any one of the above items (3) to (5), wherein the amine is trometamol.
(7) The pharmaceutical composition according to any one of the above items (3) to (6), wherein the amino acid is ε-aminocaproic acid, glycine or L-glutamine.
(8) The pharmaceutical composition according to any one of the above items (3) to (5), wherein the buffering agent is citric acid or a salt thereof and trometamol or a salt thereof.
(9) The pharmaceutical composition according to any one of the above items (1) to (8), wherein the amount of (+)-11-[2-[2-[(diethylamino)methyl]-1-piperidinyl]acetyl]-5,11-dihydro-6H-pyrido[2,3-b][1,4]benzodiazepin-6-one or a salt thereof in the pharmaceutical composition is 0.0001 to 5% (w/v).
(10) The pharmaceutical composition according to any one of the above items (3) to (9), wherein the amount of the buffering agent in the pharmaceutical composition is 0.001 to 10% (w/v).
(11) The pharmaceutical composition according to any one of the above items (3) to (10), wherein the amount of the buffering agent in the pharmaceutical composition is 0.1 to 20000 parts by weight or 0.01 to 1000 parts by weight per part by weight of (+)-11-[2-[2-[(diethylamino)methyl]-1-piperidinyl]acetyl]-5,11-dihydro-6H-pyrido[2,3-b][1,4]benzodiazepin-6-one or a salt thereof.
(12) The pharmaceutical composition according to any one of the above items (1) to (11) for ocular topical administration.
(13) The pharmaceutical composition according to the above item (12), wherein the ocular topical administration is instillation administration, conjunctival sac administration, intravitreal administration, sub-conjunctival administration or sub-Tenon's administration.
(14) The pharmaceutical composition according to any one of the above items (1) to (13) which is an eye drop, an eye gel or an injection.
(15) The pharmaceutical composition according to any one of the above items (1), (2) and (4) to (14) stored at 50°C or less.
(16) A method of stabilizing (+)-11-[2-[2-[(diethylamino)methyl]-1-piperidinyl]acetyl)-5,11-dihydro-6H-pyrido[2,3-b][1,4]benzodiazepin-6-one or a salt thereof in a pharmaceutical composition comprising the same, which comprises adjusting the pH of the pharmaceutical composition to 6.5 or less.
(17) A method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof in a pharmaceutical composition comprising the same, which comprises adding one or more buffering agents selected from the group consisting of citric acid, acetic acid, malic acid, an amine, an amino acid or a salt thereof into the pharmaceutical composition.
(18) A method of inhibiting the production of 5,11-dihydro-6H-pirido[2,3-b] [1,4]benzodiazepin-6-one, which comprises adjusting the pH of a pharmaceutical composition comprising (+)-11-[2-[2-[(diethylamino)methyl]-1-piperidinyl]acetyl]-5,11-dihydro-6H-pyrido[2,3-b][1,4]benzodiazepin-6-one or a salt thereof to 6.5 or less.
(19) A method of inhibiting the photolytic degradation of (+)-11-[2-[2-[(diethylamino)methyl]-1-piperidinyl]acetyl]-5,11-dihydro-6H-pyrido[2,3-b][1,4]benzodiazepin-6-one or a salt thereof, which comprises adding one or more buffering agents selected from the group consisting of citric acid, acetic acid, malic acid, an amine, an amino acid or a salt thereof into a pharmaceutical composition comprising (+)-11-[2-[2-[(diethylamino)methyl]-1-piperidinyl]acetyl]-5,11-dihydro-6H-pyrido[2,3-b] [1,4]benzodiazepin-6-one or a salt thereof.
(20) The method according to the above item (16) or (18) which further comprises storing the pharmaceutical composition at 50°C or less.
(21) The method according to the above item (17) or (19), wherein the buffering agent is citric acid or a salt thereof and trometamol or a salt thereof.

Each feature of the above (1) to (21) may be optionally selected and combined two or more.

### (EFFECTS OF THE INVENTION)

According to the present invention, the production of a hydrolysate of the compound of the present invention in a pharmaceutical composition can be inhibited. Hence, a pharmaceutical composition stabilized over a long period can be provided. According to the present invention, a method of stabilizing the compound of the present invention in a pharmaceutical composition over a long period can be also provided.

In addition, the pharmaceutical composition and the stabilization method of the present invention can provide a stable formulation comprising (+)-11-[2-[2-[(diethylamino)methyl]-1-piperidinyl]acetyl]-5,11-dihydro-6H-pyrido[2,3-b] [1,4]benzodiazepin-6-one or a salt thereof. Hence, the formulation has superior storage stability and safety and thus can be used and stored for a long period.

### DETAILED DESCRIPTION OF THE PREFERRED EMBODIMENTS

Hereinafter, the present invention is illustrated in detail.

The present invention provides a stable pharmaceutical composition comprising (+)-11-[2-[2-[(diethylamino)methyl]-1-piperidinyl]acetyl]-5,11-dihydro-6H-pyrido[2,3-b][1,4]benzodiazepin-6-one (hereinafter also referred to as "AFDX0250") or a salt thereof.

As used herein, the term "(+)-11-[2-[2-[(diethylamino)methyl]-1-piperidinyl]acetyl]-5,11-dihydro-6H-pyrido[2,3-b][1,4]benzodiazepin-6-one" or "AFDX0250" is a compound of the following formula (CAS Registration No. 121029-35-4): 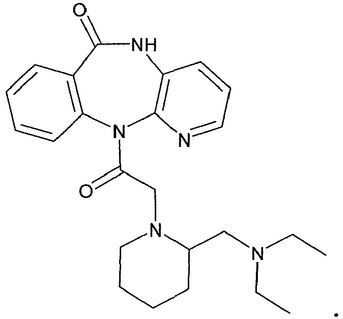 Also, in the present invention, (+)-11-[2-[2-[(diethylamino)methyl]-1-piperidinyl]acetyl]-5,11-dihydro-6H-pyrido[2,3-b][1,4]benzodiazepin-6-one means (R)-11-[2-[2-[(diethylamino)methyl]-1-piperidinyl]acetyl]-5,11-dihydro-6H-pirido[2,3-b][1,4]benzodiazepin-6-one and is represented by the following formula:

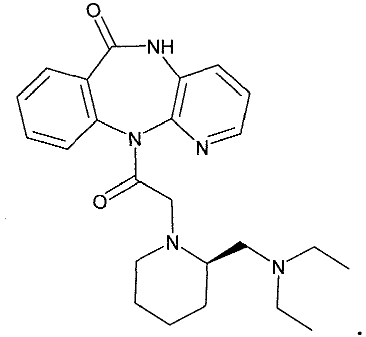

In the present invention, (+)-11-[2-[2-[(diethylamino)methyl]-1-piperidinyl]acetyl]-5,11-dihydro-6H-pyrido[2,3-b][1,4]benzodiazepin-6-one may be in a salt form, and it is not particularly limited as long as the salt is a pharmaceutically acceptable salt. Examples thereof include a salt with an inorganic acid such as hydrochloric acid, hydrobromic acid, hydroiodic acid, nitric acid, sulfuric acid and phosphoric acid; a salt with an organic acid such as acetic acid, oxalic acid, fumaric acid, maleic acid, succinic acid, malic acid, citric acid, tartaric acid, adipic acid, gluconic acid, glucoheptonic acid, glucuronic acid, terephthalic acid, methanesulfonic acid, alanine, lactic acid, hippuric acid, 1,2-ethanedisulfonic acid, isethionic acid, lactobionic acid, oleic acid, gallic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid and sulfosalicylic acid; a salt with a metal such as sodium, potassium, calcium and magnesium; a salt with an inorganic compound such as ammonia; and a salt with an organic amine such as triethylamine and guanidine.

In the pharmaceutical composition of the present invention, (+)-11-[2-[2-[(diethylamino)methyl]-1-piperidinyl]acetyl]-5,11-dihydro-6H-pyrido[2,3-b] [1,4]benzodiazepin-6-one or a salt thereof may be in the form of a hydrate or a solvate.

In the present invention, (+)-11-[2-[2-[(diethylamino)methyl]-1-piperidinyl]acetyl]-5,11-dihydro-6H-pyrido[2,3-b][1,4]benzodiazepin-6-one or a salt thereof may be prepared according to a well-known method in the organic chemistry field or may be also obtained as a commercially available product. For example, (+)-11-[2-[2-[(diethylamino)methyl]-1-piperidinyl]acetyl]-5,11-dihydro-6H-pyrido[2,3-b][1,4]benzodiazepin-6-one can be prepared according to the step described in J. Med. Chem., 32(8), 1718-24, 1989.

The amount of (+)-11-[2-[2-[(diethylamino)methyl]-1-piperidinyl]acetyl]-5,11-dihydro-6H-pyrido[2,3-b][1,4]benzodiazepin-6-one or a salt thereof in the pharmaceutical composition of the present invention is not particularly limited. The lower limit thereof is preferably 0.0001% (w/v), more preferably 0.0003% (w/v), furthermore preferably 0.0005% (w/v), even more preferably 0.001% (w/v), particularly preferably 0.0013% (w/v) particularly more preferably 0.0015% (w/v), particularly furthermore preferably 0.0017% (w/v), and most preferably 0.002% (w/v). The upper limit thereof is preferably 5% (w/v), more preferably 3% (w/v), furthermore preferably 2% (w/v), even more preferably 1% (w/v), particularly preferably 0.5% (w/v), particularly more preferably 0.2% (w/v), and most preferably 0.1% (w/v). From the viewpoint of preventing the precipitation of degradation products of (+)-11-[2-[2-[(diethylamino)methyl]-1-piperidinyl]acetyl]-5,11-dihydro-6H-pyrido[2,3-b] [1,4]benzodiazepin-6-one or a salt thereof, the upper limit thereof is preferably 0.5% (w/v), more preferably 0.2% (w/v), and most preferably 0.1% (w/v). The amount of (+)-11-[2-[2-[(diethylamino)methyl]-1-piperidinyl]acetyl]-5,11-dihydro-6H-pyrido[2,3-b] [1,4]benzodiazepin-6-one or a salt thereof in the pharmaceutical composition is preferably 0.0001 to 5% (w/v), more preferably 0.0003 to 3% (w/v), furthermore preferably 0.0005 to 2% (w/v), even more preferably 0.001 to 1% (w/v), particularly preferably 0.0013 to 0.5% (w/v), particularly more preferably 0.0015 to 0.2% (w/v), particularly furthermore preferably 0.0017 to 0.1% (w/v), and most preferably 0.002 to 0.1% (w/v).

As used herein, the term "% (w/v) " means the mass (g) of an object ingredient in 100 mL of the pharmaceutical composition of the present invention. When the pharmaceutical composition of the present invention comprises a salt of (+)-11-[2-[2-[(diethylamino)methyl]-1-piperidinyl]acetyl]-5,11-dihydro-6H-pyrido[2,3-b] [1,4]benzodiazepin-6-one, the value may mean the amount of the salt of (+)-11-[2-[2-[(diethylamino)methyl]-1-piperidinyl]acetyl]-5,11-dihydro-6H-pyrido[2,3-b][1,4]benzodiazepin-6-one or the amount of (+)-11-[2-[2-[(diethylamino)methyl]-1-piperidinyl]acetyl]-5,11-dihydro-6H-pyrido[2,3-b][1,4]benzodiazepin-6-one. In addition, when the pharmaceutical composition of the present invention comprises (+)-11-[2-[2-[(diethylamino)methyl]-1-piperidinyl]acetyl]-5,11-dihydro-6H-pyrido[2,3-b][1,4]benzodiazepin-6-one or a salt thereof in the form of a hydrate or a solvate, the value may mean the amount of (+)-11-[2-[2-[(diethylamino)methyl]-1-piperidinyl]acetyl]-5,11-dihydro-6H-pyrido[2,3-b][1,4]benzodiazepin-6-one or a salt thereof in the form of a hydrate or a solvate or the amount of (+)-11-[2-[2-[(diethylamino)methyl]-1-piperidinyl]acetyl)-5,11-dihydro-6H-pyrido[2,3-b][1,4]benzodiazepin-6-one or a salt thereof. Hereinafter, the same shall apply to the followings unless otherwise specified.

In an embodiment of the pharmaceutical composition of the present invention, the pH thereof is 6.5 or less, preferably to 4 to 6.5, more preferably 5 to 6.3, furthermore preferably 5.3 to 6.2, and most preferably 5.5 to 6. As long as the pH thereof is within the above ranges, the pharmaceutical composition of the present invention inhibits the hydrolysis of (+)-11-[2-[2-[(diethylamino)methyl]-1-piperidinyl]acetyl]-5,11-dihydro-6H-pyrido[2,3-b] [1,4]benzodiazepin-6-one and thus can inhibit the production of 5,11-dihydro-6H-pirido[2,3-b] [1,4]benzodiazepin-6-one which is a hydrolysate thereof.

In an embodiment of the pharmaceutical composition of the present invention, the pharmaceutical composition of the present invention comprises a buffering agent. The buffering agent in the pharmaceutical composition of the present invention is one or more buffering agents selected from the group consisting of citric acid, malic acid, an amine, an amino acid and a salt thereof. The pharmaceutical composition of the present invention becomes stable to light by the addition of the buffering agents. The buffering agent of the present invention comprises citric acid or a salt thereof and is preferably used in combination with any of acetic acid or a salt thereof, malic acid or a salt thereof, an amine or a salt thereof, or an amino acid or a salt thereof from the viewpoint of further improving the light stability of (+)-11-[2-[2-[(diethylamino)methyl]-1-piperidinyl]acetyl]-5,11-dihydro-6H-pyrido[2,3-b] [1,4]benzodiazepin-6-one or a salt thereof.

Examples of the salt of citric acid include sodium citrate, disodium citrate, trisodium citrate, and others.

Examples of the salt of acetic acid include sodium acetate, potassium acetate, and others.

Malic acid may be in the form of racemate or enantiomer (L-body or D-body). Examples of the salt thereof include sodium malate, disodium malate, and others.

Examples of the amine include trometamol, monoethanolamine, diethanolamine, triethanolamine, meglumine, and others. Examples of the salt thereof include hydrochloride salt thereof, acetate salt thereof, and others. The amine of the present invention is preferably trometamol, and examples of the salt thereof include trometamol hydrochloride, and others.

Examples of the amino acid include ε-aminocaproic acid, glycine, alanine, valine, leucine, serine, threonine, methionine, threonine, glutamine, glutamic acid, asparagine, aspartic acid, arginine, lysine, histidine, and others. When they have optical isomers, they may be in the form of racemate or enantiomer (L-body or D-body). Examples of the salt thereof include hydrochloride salt thereof, acetate salt thereof, and others. The amino acid of the present invention is preferably ε-aminocaproic acid, glycine, L-glutamine, and examples of the salt thereof include glycine hydrochloride, L-glutamine hydrochloride, and others.

In the present invention, the buffering agent may be in the form of a hydrate or a solvate. Examples thereof include citric acid hydrate, sodium citrate hydrate, and others.

The lower limit of the amount of the buffering agent in the pharmaceutical composition of the present invention is preferably 0.001% (w/v), more preferably 0.005% (w/v), furthermore preferably 0.01% (w/v), even more preferably 0.05% (w/v), particularly preferably 0.1% (w/v), and most preferably 0.2% (w/v). The upper limit of the amount of the buffering agent in the pharmaceutical composition of the present invention is, for example, 20% (w/v), preferably 10% (w/v), more preferably 5% (w/v), furthermore preferably 4% (w/v), even more preferably 3% (w/v), particularly preferably 2% (w/v), and most preferably 1.5% (w/v). The amount of the buffering agent in the pharmaceutical composition of the present invention is, for example, 0.001 to 20% (w/v), preferably 0.001 to 10% (w/v), more preferably 0.005 to 5% (w/v), furthermore preferably 0.01 to 4% (w/v), even more preferably 0.05 to 3% (w/v), particularly preferably 0.1 to 2% (w/v), and most preferably 0.2 to 1.5% (w/v). For example, the amount of the buffering agent in the pharmaceutical composition of the present invention may be 0.001% (w/v), 0.002% (w/v), 0.005% (w/v), 0.01% (w/v), 0.02% (w/v), 0.05% (w/v), 0.1% (w/v)0.2% (w/v), 1.5% (w/v), 2% (w/v), 3% (w/v), 4% (w/v), 5% (w/v), 6% (w/v), 8% (w/v), 10% (w/v) or 20% (w/v).

Also, in an embodiment of the pharmaceutical composition of the present invention, the lower limit of the amount of the buffering agent in the pharmaceutical composition of the present invention is preferably 0.1 part by mass, more preferably 1 part by mass, furthermore preferably 10 parts by mass, even more preferably 50 parts by mass, particularly preferably 100 parts by mass, and most preferably 500 parts by mass per part by mass of (+)-11-[2-[2-[(diethylamino)methyl]-1-piperidinyl]acetyl]-5,11-dihydro-6H-pyrido[2,3-b] [1,4]benzodiazepin-6-one or a salt thereof. In another embodiment of the pharmaceutical composition of the present invention, the lower limit of the amount of the buffering agent in the pharmaceutical composition of the present invention is preferably 0.01 part by mass, more preferably 0.03 part by mass, furthermore preferably 0.05 part by mass, even more preferably 0.1 part by mass, particularly preferably 0.3 part by mass, and most preferably 0.5 part by mass per part by mass of (+)-11-[2-[2-[(diethylamino)methyl]-1-piperidinyl]acetyl]-5,11-dihydro-6H-pyrido[2,3-b][1,4]benzodiazepin-6-one or a salt thereof. Also, in an embodiment of the pharmaceutical composition of the present invention, the upper limit of the amount of the buffering agent in the pharmaceutical composition of the present invention is preferably 20000 parts by mass, more preferably 10000 parts by mass, furthermore preferably 5000 parts by mass, even more preferably 3000 parts by mass, particularly preferably 2000 parts by mass, and most preferably 1000 parts by mass per part by mass of (+)-11-[2-[2-[(diethylamino)methyl]-1-piperidinyl]acetyl]-5,11-dihydro-6H-pyrido[2,3-b][1,4]benzodiazepin-6-one or a salt thereof. In another embodiment of the pharmaceutical composition of the present invention, the upper limit of the amount of the buffering agent in the pharmaceutical composition of the present invention is preferably 1000 parts by mass, more preferably 100 parts by mass, furthermore preferably 50 parts by mass, even more preferably 10 parts by mass, particularly preferably 6 parts by mass, and most preferably 3 parts by mass per part by weight of (+)-11-[2-[2-[(diethylamino)methyl]-1-piperidinyl]acetyl]-5,11-dihydro-6H-pyrido[2,3-b] [1,4]benzodiazepin-6-one or a salt thereof. Also, in an embodiment of the pharmaceutical composition of the present invention, the amount of the buffering agent in the pharmaceutical composition of the present invention is preferably 0.1 to 20000 parts by mass, more preferably 1 to 10000 parts by mass, furthermore preferably 10 to 5000 parts by mass, even more preferably 50 to 3000 parts by mass, particularly preferably 100 to 2000 parts by mass, and most preferably 500 to 1000 parts by mass per part by mass of (+)-11-[2-[2-[(diethylamino)methyl]-1-piperidinyl]acetyl]-5,11-dihydro-6H-pyrido[2,3-b][1,4]benzodiazepin-6-one or a salt thereof. In another embodiment of the pharmaceutical composition of the present invention, the amount of the buffering agent in the pharmaceutical composition of the present invention is preferably 0.01 to 1000 parts by mass, more preferably 0.03 to 100 parts by mass, furthermore preferably 0.05 to 50 parts by mass, even more preferably 0.1 to 10 parts by mass, particularly preferably 0.3 to 6 parts by mass, and most preferably 0.5 to 3 parts by mass per part by mass of (+)-11-[2-[2-[(diethylamino)methyl]-1-piperidinyl]acetyl]-5,11-dihydro-6H-pyrido[2,3-b] [1,4]benzodiazepin-6-one or a salt thereof.

In an embodiment of the pharmaceutical composition of the present invention, the pharmaceutical composition of the present invention comprises citric acid or a salt thereof and trometamol or a salt thereof as the buffering agent. In such case, the lower limits of the amounts of citric acid or a salt thereof and trometamol or a salt thereof in the pharmaceutical composition of the present invention are preferably 0.0005% (w/v), more preferably 0.001% (w/v), furthermore preferably 0.005% (w/v), even more preferably 0.01% (w/v), particularly preferably 0.05% (w/v), and most preferably 0.1% (w/v), respectively. The upper limits of the amounts of citric acid or a salt thereof and trometamol or a salt thereof in the pharmaceutical composition of the present invention are preferably 10% (w/v), more preferably 5% (w/v), furthermore preferably 4% (w/v), even more preferably 3% (w/v), particularly preferably 2% (w/v), and most preferably 1.5% (w/v), respectively. The amounts of citric acid or a salt thereof and trometamol or a salt thereof in the pharmaceutical composition of the present invention are preferably 0.0005 to 10% (w/v), more preferably 0.001 to 5% (w/v), furthermore preferably 0.005 to 4% (w/v), even more preferably 0.01 to 3% (w/v), particularly preferably 0.05 to 2% (w/v), and most preferably 0.1 to 1.5% (w/v), respectively. Also, the lower limits of the amounts of citric acid or a salt thereof and trometamol or a salt thereof in the pharmaceutical composition of the present invention are preferably 0.005 part by mass, more preferably 0.01 part by mass, furthermore preferably 0.03 part by mass, even more preferably 0.05 part by mass, particularly preferably 0.1 part by mass, and most preferably 0.3 part by mass per part by mass of (+)-11-[2-[2-[(diethylamino)methyl]-1-piperidinyl]acetyl]-5,11-dihydro-6H-pyrido[2,3-b] [1,4]benzodiazepin-6-one or a salt thereof, respectively. The upper limits of the amounts of citric acid or a salt thereof and trometamol or a salt thereof in the pharmaceutical composition of the present invention is preferably 100 parts by mass, more preferably 50 parts by mass, furthermore preferably 10 parts by mass, even more preferably 6 parts by mass, particularly preferably 3 parts by mass, and most preferably 2 parts by mass per part by mass of (+)-11-[2-[2-[(diethylamino)methyl]-1-piperidinyl]acetyl]-5,11-dihydro-6H-pyrido[2,3-b][1,4]benzodiazepin-6-one or a salt thereof, respectively. The amounts of citric acid or a salt thereof and trometamol or a salt thereof in the pharmaceutical composition of the present invention are preferably 0.005 to 100 parts by mass, more preferably 0.01 to 50 parts by mass, furthermore preferably 0.03 to 10 parts by mass, even more preferably 0.05 to 6 parts by mass, particularly preferably 0.1 to 3 parts by mass, and most preferably 0.3 to 2 parts by mass per part by mass of (+)-11-[2-[2-[(diethylamino)methyl]-1-piperidinyl)acetyl]-5,11-dihydro-6H-pyrido[2,3-b][1,4]benzodiazepin-6-one or a salt thereof, respectively.

The pharmaceutical composition of the present invention may comprise a pharmaceutically acceptable active ingredient other than (+)-11-[2-[2-[(diethylamino)methyl]-1-piperidinyl]acetyl]-5,11-dihydro-6H-pyrido[2,3-b][1,4]benzodiazepin-6-one or a salt thereof, for example, an active ingredient with an effect of treating an eye disease such as myopia as long as the effect of the present invention is not inhibited. Also, the pharmaceutical composition of the present invention may comprise no pharmaceutically acceptable active ingredient other than (+)-11-[2-[2-[(diethylamino)methyl]-1-piperidinyl]acetyl]-5,11-dihydro-6H-pyrido[2,3-b][1,4]benzodiazepin-6-one or a salt thereof.

The pharmaceutical composition of the present invention may comprise an additive as appropriate. Examples of the additive include a surfactant, a tonicity agent, a stabilizing agent, a preservative, an antioxidant, a high molecular weight polymer, a pH adjuster and a base.

In the pharmaceutical composition of the present invention, a surfactant available as pharmaceutical additives such as a cationic surfactant, an anionic surfactant and a non-ionic surfactant may be added as appropriate.

Examples of the anionic surfactant include phosphorous lipid and others. Examples of the phosphorous lipid include lecithin and others.

Examples of the cationic surfactant include alkylamine salt, alkylamine-polyoxyethylene additive, fatty acid triethanolamine monoester salt, acylaminoethyldiethylamine salt, fatty acid-polyamine conjugate, alkyl trimethyl ammonium salt, dialkyl dimethyl ammonium salt, alkyl dimethyl benzyl ammonium salt, alkyl pyridinium salt, acylaminoalkyl-type ammonium salt, acylaminoalkyl pyridinium salt, diacyloxyethyl ammonium salt, alkyl imidazoline, 1-acylaminoethyl-2-alkyl imidazoline, 1-hydroxylethyl-2-alkyl imidazoline, and others. Examples of the alkyl dimethyl benzyl ammonium salt include benzalkonium chloride, cetalkonium chloride, and others.

Examples of the non-ionic surfactant include polyoxyethylene fatty acid ester, polyoxyethylene sorbitan fatty acid ester, polyoxyethylene hydrogenated castor oil, polyoxyethylene castor oil, polyoxyethylene polyoxypropylene glycol, sucrose fatty acid ester, vitamin E TPGS, and others.

Examples of the polyoxyethylene fatty acid ester include Polyoxyl 40 stearate and others.

Examples of the polyoxyethylene sorbitan fatty acid ester include Polysorbate 80, Polysorbate 65, Polysorbate 60, Polysorbate 40, polyoxyethylene sorbitan monolaurate, polyoxyethylene sorbitan trioleate, and others.

As the polyoxyethylene hydrogenated castor oil, various polyoxyethylene hydrogenated castor oils with different numbers of ethylene oxide polymerizations may be used, and the number of ethylene oxide polymerizations is preferably 10 to 100, more preferably 20 to 80, particularly 40 to 70, and most preferably 60. Specific examples of the polyoxyethylene hydrogenated castor oil include polyoxyethylene hydrogenated castor oil 10, polyoxyethylene hydrogenated castor oil 40, polyoxyethylene hydrogenated castor oil 50, polyoxyethylene hydrogenated castor oil 60, and others.

As the polyoxyethylene castor oil, various polyoxyethylene castor oils with different numbers of ethylene oxide polymerizations may be used, and the number of ethylene oxide polymerizations is preferably 5 to 100, more preferably 20 to 50, particularly preferably 30 to 40, and most preferably 35. Specific examples of the polyoxyethylene castor oil include polyoxyl 5 castor oil, polyoxyl 9 castor oil, polyoxyl 15 castor oil, polyoxyl 35 castor oil, polyoxyl 40 castor oil, and others.

Examples of the polyoxyethylene polyoxypropylene glycol include polyoxyethylene (160) polyoxypropylene (30) glycol, polyoxyethylene (42) polyoxypropylene (67) glycol, polyoxyethylene (54) polyoxypropylene (39) glycol, polyoxyethylene (196) polyoxypropylene (67) glycol, polyoxyethylene (20) polyoxypropylene (20) glycol, and others.

Examples of the sucrose fatty acid ester include sucrose stearate and others.

The vitamin E TPGS also means tocopherol polyethylene glycol 1000 succinate.

The amount of the surfactant in the pharmaceutical composition of the present invention may be suitably varied depending on, for example, the type of the surfactant. The amount thereof is preferably 0.001 to 10% (w/v), more preferably 0.01 to 5% (w/v), furthermore preferably 0.05 to 3% (w/v), and most preferably 0.1 to 2% (w/v).

In the pharmaceutical composition of the present invention, a tonicity agent available as pharmaceutical additives may be added as appropriate to adjust the osmotic pressure thereof. The osmotic pressure ratio of the pharmaceutical composition of the present invention is, for example, 1 and is not particularly limited as long as it is within pharmaceutically acceptable ranges. Examples thereof include an ionic tonicity agent, a non-ionic tonicity agent, and others.

Examples of the ionic tonicity agent include sodium chloride, potassium chloride, calcium chloride, magnesium chloride, and others. Examples of the non-ionic tonicity agent include glycerin, propylene glycol, sorbitol, mannitol, and others.

The amount of the tonicity agent in the pharmaceutical composition of the present invention may be suitably varied depending on, for example, the type of the tonicity agent. The amount thereof is preferably 0.01 to 10% (w/v), more preferably 0.02 to 7% (w/v), furthermore preferably 0.1 to 5% (w/v), particularly preferably 0.3 to 4% (w/v), and most preferably 0.5 to 3% (w/v).

In the pharmaceutical composition of the present invention, a stabilizing agent available as pharmaceutical additives may be added as appropriate. Examples of the stabilizing agent include edetic acid, sodium edetate, and others.

The amount of the stabilizing agent in the pharmaceutical composition of the present invention may be suitably varied depending on, for example, the type of the stabilizing agent. The amount thereof is preferably 0.001 to 10% (w/v), more preferably 0.01 to 5% (w/v), furthermore preferably 0.05 to 3% (w/v), and most preferably 0.1 to 2% (w/v).

In the pharmaceutical composition of the present invention, a preservative available as pharmaceutical additives may be added as appropriate. Examples of the preservative include benzalkonium chloride, benzalkonium bromide, benzethonium chloride, sorbic acid, potassium sorbate, methyl paraoxybenzoate, propyl paraoxybenzoate, chlorobutanol, and others.

The amount of the preservative in the pharmaceutical composition of the present invention may be suitably varied depending on, for example, the type of the preservative. The amount thereof is preferably 0.0001 to 1% (w/v), more preferably 0.0005 to 0.1% (w/v), furthermore preferably 0.001 to 0.05% (w/v), and most preferably 0.002 to 0.01% (w/v) .

In the pharmaceutical composition of the present invention, an antioxidant available as pharmaceutical additives may be added as appropriate. Examples of the antioxidant include tocopherol, dibutylhydroxytoluene, butylhydroxyanisole, sodium erythorbate, propyl gallate, sodium sulfite, and others.

The amount of the antioxidant in the pharmaceutical composition of the present invention may be suitably varied depending on, for example, the type of the antioxidant. The amount thereof is preferably 0.0001 to 1% (w/v), more preferably 0.0005 to 0.1% (w/v), furthermore preferably 0.001 to 0.02% (w/v) and most preferably 0.005 to 0.010% (w/v).

In the pharmaceutical composition of the present invention, a high molecular weight polymer available as pharmaceutical additives may be added as appropriate. Examples of the high molecular weight polymer include methyl cellulose, ethyl cellulose, hydroxymethyl cellulose, hydroxyethyl cellulose, hydroxypropyl cellulose, hydroxyethyl methyl cellulose, hydroxypropyl methyl cellulose (hypromellose), carboxymethyl cellulose, sodium carboxymethyl cellulose, hydroxypropyl methyl cellulose acetate succinate, hydroxypropyl methyl cellulose phthalate, carboxymethyl ethyl cellulose, cellulose acetate phthalate, polyvinylpyrrolidone, polyvinyl alcohol, carboxyvinyl polymer, polyethylene glycol, and others. The high molecular weight polymer of the present invention is preferably hydroxypropyl methyl cellulose (hypromellose).

The amount of the high molecular weight polymer in the pharmaceutical composition of the present invention may be suitably varied depending on, for example, the type of the high molecular weight polymer. The amount thereof is preferably 0.001 to 5% (w/v), more preferably 0.01 to 1% (w/v), and furthermore preferably 0.1 to 0.5% (w/v).

In the pharmaceutical composition of the present invention, a pH adjuster available as pharmaceutical additives may be added as appropriate as long as the pH thereof is 6.5 or less. Examples of the pH adjuster include hydrochloric acid, sodium hydroxide, potassium hydroxide, and others.

The amount of the pH adjuster in the pharmaceutical composition of the present invention may be suitably varied depending on, for example, the type of the pH adjuster. The amount thereof is preferably 0.001 to 5% (w/v), more preferably 0.01 to 1% (w/v), and furthermore preferably 0.1 to 0.5% (w/v).

In the pharmaceutical composition of the present invention, a base available as pharmaceutical additives may be added as appropriate. Examples of the base include water, purified water, saline, and others.

The pharmaceutical composition of the present invention is preferably an aqueous pharmaceutical composition. On the other hand, it may be a non-aqueous pharmaceutical composition.

In an embodiment of the pharmaceutical composition of the present invention, the pharmaceutical composition comprises no surfactant. In an embodiment of the pharmaceutical composition of the present invention, the pharmaceutical composition comprises no tonicity agent. In an embodiment of the pharmaceutical composition of the present invention, the pharmaceutical composition comprises no stabilizing agent. In an embodiment of the pharmaceutical composition of the present invention, the pharmaceutical composition comprises no preservative. In an embodiment of the pharmaceutical composition of the present invention, the pharmaceutical composition comprises no antioxidant. In an embodiment of the pharmaceutical composition of the present invention, the pharmaceutical composition comprises no high molecular weight polymer. In an embodiment of the pharmaceutical composition of the present invention, the pharmaceutical composition comprises no pH adjuster.

When the pharmaceutical composition of the present invention is stored at 50°C or less, the production of a hydrolysate of the compound of the present invention can be inhibited. Hence, the pharmaceutical composition has superior stability when the temperature is 50°C or less. Also, when the pharmaceutical composition of the present invention is stored at lower temperature, preferably 30°C or less, more preferably 25°C or less, furthermore preferably 10°C or less, even more preferably, 5°C or less, even furthermore preferably 0°C or less, particularly preferably -10°C or less, and most preferably -20°C or less, it is kept in a stable condition. The lowest temperature for storing the pharmaceutical composition of the present invention is preferably -80°C, more preferably -60°C, furthermore preferably -50°C, particularly preferably -40°C, and most preferably -30°C. The temperature for storing the pharmaceutical composition of the present invention is, for example, -80°C to 50°C, preferably -80°C to 30°C, more preferably -60°C to 25°C, furthermore preferably -50°C to 10°C, even more preferably -40°C to 5°C, even furthermore preferably -30°C to 0°C, particularly preferably -30°C to - 10°C, and most preferably -30°C to -20°C.

The pharmaceutical composition of the present invention may be administered orally or parenterally. Examples of the administration route include oral administration, intravenous administration, transdermal administration, and ocular topical administration (e.g., instillation administration, conjunctival sac administration, intravitreal administration, sub-conjunctival administration, sub-Tenon's administration), and others.

The dosage form of the pharmaceutical composition of the present invention is not particularly limited as long as it is available as a pharmaceutical product. Examples of the dosage form thereof include an eye drop, an eye gel, an injection, and others. The dosage form of the pharmaceutical composition of the present invention is particularly preferably an eye drop. When the pharmaceutical composition of the present invention is used as non-aqueous pharmaceutical composition, it may be formulated as a non-aqueous formulation.

They may be prepared according to a commonly-used method in the art.

The pharmaceutical composition of the present invention may be filled and stored in a container made of various materials.

In addition, the present invention provides a method of stabilizing (+)-11-[2-[2-[(diethylamino)methyl]-1-piperidinyl]acetyl]-5,11-dihydro-6H-pyrido[2,3-b] [1,4]benzodiazepin-6-one or a salt thereof in the pharmaceutical composition. The above description for the pharmaceutical composition of the present invention is applied to the method of stabilizing (+)-11-[2-[2-[(diethylamino)methyl]-1-piperidinyl)acetyl]-5,11-dihydro-6H-pyrido[2,3-b][1,4]benzodiazepin-6-one or a salt thereof in the pharmaceutical composition of the present invention.

The method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof in the pharmaceutical composition of the present invention encompasses a method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof in a pharmaceutical composition comprising the same, which comprises adjusting the pH of the pharmaceutical composition to 6.5 or less. The method inhibits the hydrolysis of (+)-11-[2-[2-[(diethylamino)methyl]-1-piperidinyl]acetyl]-5,11-dihydro-6H-pyrido[2,3-b][1,4]benzodiazepin-6-one and thus can inhibit the production of 5,11-dihydro-6H-pirido[2,3-b][1,4]benzodiazepin-6-one which is a hydrolysate thereof.

The method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof in the pharmaceutical composition of the present invention also encompasses a method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof in a pharmaceutical composition comprising the same, which comprises adding one or more buffering agents selected from the group consisting of citric acid, acetic acid, malic acid, an amine, an amino acid and a salt thereof into the pharmaceutical composition. The method inhibits the degradation of (+)-11-[2-[2-[(diethylamino)methyl]-1-piperidinyl]acetyl]-5,11-dihydro-6H-pyrido[2,3-b] [1,4]benzodiazepin-6-one or a salt thereof under the light irradiation and thus (+)-11-[2-[2-[(diethylamino)methyl]-1-piperidinyl]acetyl]-5,11-dihydro-6H-pyrido[2,3-b] [1,4]benzodiazepin-6-one or a salt thereof becomes stable to light.

### EXAMPLES

Hereinafter, the present invention is illustrated in Formulation Examples and Test Results in order to make it easy to understand the present invention. The present invention, however, is not intended to be limited thereto by any means.

### Formulation Examples

Typical examples of formulations comprising the pharmaceutical composition of the present invention are shown below. The amount of each ingredient formulated in the following formulation examples (% (w/v)) is the amount (g) in 100 mL of the composition.

**[Table 1]**

| Formulation Examples %(w/v) | 1 | 2 | 3 | 4 | 5 | 6 | 7 | 8 | 9 | 10 |
|---|---|---|---|---|---|---|---|---|---|---|
| AFDX0250 | 0.1 | 1.0 | 0.01 | 0.2 | 0.5 | 0.5 | 0.1 | 0.2 | 0.001 | 1.5 |
| Sodium acetate hydrate | 0.15 | | | 0.5 | | 0.25 | | | | |
| Sodium citrate hydrate | | | 0.2 | | 0.3 | | 0.15 | 0.5 | | 0.3 |
| ε-Aminocapric acid | | 0.3 | | | | 0.1 | 0.1 | | 0.25 | |
| Sodium dihydrogen phosphate hydrate | | | | 0.25 | | | | | 0.2 | |
| Trometamol | 0.3 | | 0.3 | | | 0.5 | | | | 1.5 |
| Boric acid | | 0.1 | | | 0.2 | | 0.5 | | | |
| Sodium chloride | | q.s. | q.s. | | | q.s. | | | | q.s. |
| Mannitol | q.s. | | | | q.s. | | q. s. | | | |
| Concentrated glycerin | | | | q.s. | | | | q.s. | q.s. | |
| Sodium hydroxide | q.s. | q.s. | q.s. | q.s. | q.s. | q.s. | q.s. | q.s. | q.s. | q.s. |
| Dilute hydrochloric acid | q.s. | q.s. | q.s. | q.s. | q.s. | q.s. | q.s. | q.s. | q.s. | q. s. |
| Purified water | q.s. | q.s. | q.s. | q.s. | q.s. | q.s. | q.s. | q.s. | q.s. | q.s. |
| pH | 6.5 | 6 | 5 | 6 | 6.5 | 5 | 6.5 | 6 | 5 | 5.5 |
| Osmotic pressure ratio | 1 | 1 | 1 | 1 | 1 | 1 | 1 | 1 | 1 | 1 |

**[Table 2]**

| Formulation Examples % (w/v) | 11 | 12 | 13 | 14 | 15 | 16 | 17 | 18 | 19 | 20 |
|---|---|---|---|---|---|---|---|---|---|---|
| AFDX0250 | 0.05 | 0.1 | 0.001 | 1.5 | 0.3 | 0.2 | 0.1 | 0.5 | 0.2 | 0.5 |
| Sodium acetate hydrate | | | 0.2 | | | | 0.15 | 0.2 | 0.25 | |
| Sodium citrate hydrate | | 0.15 | | 0.25 | 0.3 | 0.1 | | | | 1.3 |
| ε-Aminocapric acid | 0.2 | | | 0.1 | | | | | | |
| Sodium dihydrogen phosphate hydrate | | | 0.5 | | | | | | | |
| Trometamol | | 0.1 | | 0.1 | | 0.25 | | 0.8 | | |
| Boric acid | 0.25 | | | | 0.2 | | 0.5 | | | |
| Sodium chloride | | q.s. | q.s. | | | q.s. | | | q.s. | |
| Mannitol | q.s. | | | | q.s. | | | | | q.s. |
| Concentrated glycerin | | | | q.s. | | | q.s. | q.s. | | |
| Sodium hydroxide | q.s. | q.s. | q.s. | q.s. | q.s. | q.s. | q.s. | q.s. | q.s. | q.s. |
| Dilute hydrochloric acid | q.s. | q.s. | q.s. | q.s. | q.s. | q.s. | q.s. | q.s. | q.s. | q.s. |
| Purified water | q.s. | q.s. | q.s. | q.s. | q.s. | q.s. | q.s. | q.s. | q.s. | q.s. |
| pH | 6 | 6 | 5 | 6.5 | 5 | 6 | 6.5 | 5 | 5.5 | 6 |
| Osmotic pressure ratio | 1 | 1 | 1 | 1 | 1 | 1 | 1 | 1 | 1 | 1 |

**[Table 3]**

| Formulation Examples %(w/v) | 21 | 22 | 23 | 24 | 25 | 26 | 27 | 28 | 29 | 30 |
|---|---|---|---|---|---|---|---|---|---|---|
| AFDX0250 | 0.2 | 0.001 | 1.5 | 0.5 | 0.2 | 0.05 | 0.5 | 0.2 | 0.1 | 0.5 |
| Sodium acetate hydrate | | 0.2 | | | | 0.1 | | | | |
| Sodium citrate hydrate | 0.1 | | | | 0.3 | | 0.15 | | 0.25 | 0.3 |
| ε-Aminocapric acid | | | 0.25 | | | 0.15 | | | 0.1 | |
| Sodium dihydrogen phosphate hydrate | 0.25 | | | 0.15 | | | | 0.2 | | |
| Trometamol | | 0.2 | | | 0.3 | | 0.1 | | | 0.5 |
| Boric acid | | | 0.5 | 0.1 | | | | 0.2 | | |
| Sodium chloride | | | | q.s. | | q.s. | q.s. | | | |
| Mannitol | q.s. | | | | q.s. | | | q.s. | q.s. | |
| Concentrated glycerin | | q.s. | q.s. | | | | | | | q.s. |
| Sodium hydroxide | q.s. | q.s. | q.s. | q.s. | q.s. | q.s. | q.s. | q.s. | q.s. | q.s. |
| Dilute hydrochloric acid | q.s. | q.s. | q.s. | q.s. | q.s. | q.s. | q.s. | q.s. | q.s. | q.s. |
| Purified water | q.s. | q.s. | q.s. | q.s. | q.s. | q.s. | q.s. | q.s. | q.s. | q.s. |
| pH | 5.5 | 6 | 5 | 5 | 6 | 5.5 | 6 | 6.5 | 6 | 5 |
| Osmotic pressure ratio | 1 | 1 | 1 | 1 | 1 | 1 | 1 | 1 | 1 | 1 |

**[Table 4]**

| Formulation Examples %(w/v) | 31 | 32 | 33 | 34 | 35 | 36 | 37 | 38 | 39 | 40 |
|---|---|---|---|---|---|---|---|---|---|---|
| AFDX0250 | 0.1 | 0.001 | 0.2 | 1.0 | 0.1 | 0.5 | 0.3 | 0.7 | 0.1 | 0.2 |
| Sodium acetate hydrate | 0.02 | | | | 0.3 | | | 0.2 | | |
| Sodium citrate hydrate | | | 0.2 | 0.25 | | | 0.25 | | | |
| ε-Aminocapric acid | | 0.05 | | | | | 0.2 | | | |
| Sodium dihydrogen phosphate hydrate | | | | | | 0.25 | | | 0.15 | 0.3 |
| Trometamol | | | 0.4 | | 0.01 | | | | 0.3 | 0.2 |
| Boric acid | 0.5 | 0.2 | | 0.05 | | 0.2 | | 0.5 | | |
| Sodium chloride | q.s. | | | | q.s. | | q.s. | | | |
| Mannitol | | q.s. | q.s. | | | | | | q.s. | q.s. |
| Concentrated glycerin | | | | q.s. | | q.s. | | q.s. | | |
| Sodium hydroxide | q.s. | q.s. | q.s. | q.s. | q.s. | q.s. | q.s. | q.s. | q.s. | q. s. |
| Dilute hydrochloric acid | q.s. | q.s. | q.s. | q.s. | q.s. | q.s. | q.s. | q.s. | q.s. | q.s. |
| Purified water | q.s. | q.s. | q.s. | q.s. | q.s. | q.s. | q.s. | q.s. | q.s. | q.s. |
| pH | 6 | 5 | 5.5 | 6.5 | 5 | 5.5 | 6 | 6 | 6.5 | 6 |
| Osmotic pressure ratio | 1 | 1 | 1 | 1 | 1 | 1 | 1 | 1 | 1 | 1 |

**[Table 5]**

| Formulation Examples % (w/v) | 41 | 42 | 43 | 44 | 45 | 46 | 47 | 48 | 49 | 50 |
|---|---|---|---|---|---|---|---|---|---|---|
| AFDX0250 | 0.5 | 0.1 | 0.01 | 0.05 | 0.002 | 0.5 | 0.7 | 0.3 | 1.0 | 0.2 |
| Sodium acetate hydrate | | 0.15 | | | 0.3 | | | 0.1 | 0.2 | |
| Sodium citrate hydrate | 0.1 | | 0.2 | | | 0.1 | | 0.05 | | 0.15 |
| ε-Aminocapric acid | | | | | | | | | 0.05 | |
| Sodium dihydrogen phosphate hydrate | | | | 0.25 | | 0.1 | | | | 0.2 |
| Trometamol | 0.2 | | | 0.1 | 0.15 | | 0.25 | | 0.02 | |
| Boric acid | | 0.05 | 0.2 | | | | | 0.5 | | |
| Sodium chloride | | | | q.s. | | q.s. | | | q.s. | q.s. |
| Mannitol | q.s. | | | | q.s. | | | q.s. | | |
| Concentrated glycerin | | q.s. | q.s. | | | | q.s. | | | |
| Sodium hydroxide | q.s. | q.s. | q.s. | q.s. | q.s. | q.s. | q.s. | q.s. | q.s. | q.s. |
| Dilute hydrochloric acid | q.s. | q.s. | q.s. | q.s. | q.s. | q.s. | q.s. | q.s. | q.s. | q.s. |
| Purified water | q.s. | q.s. | q.s. | q.s. | q.s. | q.s. | q.s. | q.s. | q.s. | q.s. |
| pH | 5.5 | 6 | 6 | 6.5 | 5 | 5 | 5.5 | 6.5 | 6 | 5 |
| Osmotic pressure ratio | 1 | 1 | 1 | 1 | 1 | 1 | 1 | 1 | 1 | 1 |

### [Formulation Example 51]

| Eye Drop (in 100 mL) | |
|---|---|
| AFDX0250 | 0.1 g |
| Sodium citrate hydrate | 0.5 g |
| Trometamol | 0.5 g |
| Glycerin | 2.0 g |
| Dilute hydrochloric acid | Appropriate quantity |
| Sodium hydroxide | Appropriate quantity |
| Purified water | Appropriate quantity |

### [Formulation Example 52]

| Eye Drop (in 100 mL) | |
|---|---|
| AFDX0250 | 0.3 g |
| Sodium citrate hydrate | 0.25 g |
| Citric acid hydrate | 0.02 g |
| ε-Aminocaproic acid | 0.5 g |
| Sodium chloride | 0.5 g |
| Dilute hydrochloric acid | Appropriate quantity |
| Sodium hydroxide | Appropriate quantity |
| Purified water | Appropriate quantity |

### [Formulation Example 53]

| Eye Drop (in 100 mL) | |
|---|---|
| AFDX0250 | 0.5 g |
| Sodium citrate hydrate | 0.25 g |
| Citric acid hydrate | 0.02 g |
| L-Malic acid | 0.5 g |
| Sodium chloride | 0.5 g |
| Dilute hydrochloric acid | Appropriate quantity |
| Sodium hydroxide | Appropriate quantity |
| Purified water | Appropriate quantity |

A desired composition may be prepared by appropriately adjusting the types and the amounts of AFDX0250, buffering agent and additive in said Formulation Examples 1 to 53.

### 1. Stability Evaluation Test (1)

The effect of pH on the stability of a pharmaceutical composition comprising the compound of the present invention was evaluated.

### 1-1. Preparation of Test Formulation

Sodium citrate hydrate (5 g) was dissolved in purified water so as to be exactly 200 mL to prepare 2.5% sodium citrate hydrate solution. (+)-11-[2-[2-[(Diethylamino)methyl]-1-piperidinyl]acetyl]-5,11-dihydro-6H-pyrido[2,3-b][1,4]benzodiazepin-6-one (AFDX0250) (0.075 g) was dissolved in purified water so as to make exactly 150 mL to prepare 0.05% AFDX0250 solution.

The 2.5% sodium citrate hydrate solution (10 mL), the 0.05% AFDX0250 solution (10 mL) and purified water (20 mL) were mixed to prepare solutions, sodium hydroxide or dilute hydrochloric acid was added thereto to adjust the pH to 5, 5.5, 6.5 or 7, and purified water was added thereto so as to be exactly 50 mL to prepare the formulations of Example 1, Example 2, Example 4 and Comparative Example 1.

The 2.5% sodium citrate hydrate solution (20 mL), the 0.05% AFDX0250 solution (20 mL) and purified water (40 mL) were mixed to prepare a solution, sodium hydroxide or dilute hydrochloric acid was added thereto to adjust the pH to 6, and purified water was added thereto so as to be exactly 100 mL to prepare the formulation of Example 3.

### 1-2. Test Method

Each of the test formulations prepared in the above 1-1 (3 mL) was filled into a container and was stored at 25°C/40%RH for 3 months. The amount of 5,11-dihydro-6H-pirido[2,3-b][1,4]benzodiazepin-6-one (hereinafter referred to as "hydrolysate") produced by the hydrolysis of AFDX0250 in each test formulation after the 3 month storage was quantified by high performance liquid chromatography to calculate the production rate thereof (%). The production ratio of the hydrolysate (%) represents the ratio of the produced hydrolysate when the amount of AFDX0250 in the test formulation is 1000 (%).

### 1-3. Test Results and Discussion

The test results are shown in Table 6.

**[Table 6]**

| %(w/v) | Ex.1 | Ex.2 | Ex.3 | Ex.4 | Comparative Ex.1 |
|---|---|---|---|---|---|
| AFDX0250 | 0.01 | 0.01 | 0.01 | 0.01 | 0.01 |
| Sodium citrate hydrate | 0.5 | 0.5 | 0.5 | 0.5 | 0.5 |
| Sodium hydroxide | q.s. | q.s. | q.s. | q.s. | q.s. |
| Dilute hydrochloric acid | q.s. | q.s. | q.s. | q.s. | q.s. |
| Purified water | q.s. | q.s. | q.s. | q.s. | q.s. |
| pH | 5 | 5.5 | 6 | 6.5 | 7 |
| Production rate of hydrolysate(%) | 1.50 | 1.43 | 1.45 | 1.69 | 2.41 |

As clearly shown in Table 6, the formulations of Examples 1 to 4 had lower production ratios of the hydrolysate as compared to the formulation of Comparative Example 1. Hence, it was confirmed in the pharmaceutical composition of the present invention with a pH of 6.5 or less that AFDX0250 had superior stability in the pharmaceutical composition.

### 2. Stability Evaluation Test (2)

The effect of light irradiation on the stability of the pharmaceutical composition comprising the compound of the present invention was evaluated.

### 2-1. Preparation of Test Formulation

Sodium citrate hydrate (4 g) was dissolved in purified water so as to be exactly 200 mL to prepare 2% sodium citrate hydrate solution. AFDX0250 (0.02 g) was dissolved in purified water so as to be exactly 200 mL to prepare 0.01% AFDX0250 solution. Trometamol (10 g) was dissolved in purified water so as to be exactly 100 mL to prepare 10% trometamol solution. Boric acid (10 g) was dissolved in purified water so as to be exactly 200 mL to prepare 5% boric acid solution. Hypromellose (5 g) was dissolved in purified water so as to be exactly 100 mL to prepare 5% hypromellose solution.

The 2% sodium citrate hydrate solution (10 mL), the 0.01% AFDX0250 solution (10 mL) and purified water (50 mL) were mixed to prepare a solution, sodium hydroxide or dilute hydrochloric acid was added thereto to adjust the pH to 6, and then purified water was added so as to be exactly 100 mL to prepare the formulation of Example 5.

The formulations of Example 6, Example 7, Example 8, Example 9, Example 10, Example 11 and Example 12 were prepared in a similar process to the process of Example 5 except that sodium acetate hydrate (1 g), 10% trometamol solution (10 mL), ε-aminocaproic acid (1 g), glycine (1 g), L-glutamine (1 g), DL-malic acid (1 g) or 5% hypromellose solution (20 mL) was further added to the composition of Example 5. The amount of purified water to be added was suitably changed depending on the amounts of additives.

The formulations of Example 13, Example 14, Example 15, Example 16 and Example 17 were prepared in a similar process to the process of Example 7 except that sodium chloride (0.75 g), glycerin (2 g), mannitol (4 g), propylene glycol (2 g), 5% hypromellose (20 mL) was further added to the composition of Example 7. The amount of purified water to be added was suitably changed depending on the amounts of additives.

The formulations of Comparative Example 2, Comparative Example 3, Comparative Example 4 and Comparative Example 5 were prepared in a similar process to the process of Example 5 except that sodium dihydrogen phosphate hydrate (3 g), 5% boric acid solution (20 mL), L-ascorbic acid (1 g) or ethyl pyruvate (1 g) was further added to the composition of Example 5. The amount of purified water to be added was suitably changed depending on the amounts of additives.

### 2-2. Test Method

Each of the test formulations prepared in the above 2-1 (3 mL) was filled in a container and exposed to 1.2 million lux·hr of light at 1000 lux/hr or shaded with aluminum foil. The amount of (+)-11-[2-[2-[(diethylamino)methyl]-1-piperidinyl]acetyl]-5,11-dihydro-6H-pyrido[2,3-b][1,4]benzodiazepin-6-one (AFDX0250) when exposed to light or shaded was quantified by high performance liquid chromatography to calculate the residual ratio of AFDX0250 (%). The residual ratio of AFDX0250 (%) represents the ratio of AFDX0250 after light exposure or shading when the amount of AFDX0250 in the test formulation is 1000 (%).

### 2-3. Test Results and Discussion

The test results are shown in Tables 7 to 10.

**[Table 7]**

| %(w/v) | | Ex. 5 | Ex. 6 | Ex. 7 | Ex. 8 | Ex. 9 |
|---|---|---|---|---|---|---|
| AFDX0250 | | 0.001 | 0.001 | 0.001 | 0.001 | 0.001 |
| Sodium citrate hydrate | | 0.2 | 0.2 | 0.2 | 0.2 | 0.2 |
| Sodium acetate hydrate | | | 1 | | | |
| Trometamol | | | | 1 | | |
| ε-Aminocaproic acid | | | | | 1 | |
| Glycine | | | | | | 1 |
| Sodium hydroxide | | q. s. | q.s. | q.s. | q.s. | q.s. |
| Dilute hydrochloric acid | | q.s. | q.s. | q.s. | q.s. | q.s. |
| Purified water | | q.s. | q.s. | q.s. | q.s. | q.s. |
| pH | | 6 | 6 | 6 | 6 | 6 |
| Residual rate of AFDX0250 (%) | Light exposure | 82.7 | 89.9 | 98.6 | 95.7 | 90.3 |
| | Shading | 101.1 | 100.9 | 101.2 | 100.9 | 100.9 |

**[Table 8]**

| %(w/v) | | Ex.10 | Ex.11 | Ex.12 |
|---|---|---|---|---|
| AFDX0250 | | 0.001 | 0.001 | 0.001 |
| Sodium citrate hydrate | | 0.2 | 0.2 | 0.2 |
| L-Glutamine | | 1 | | |
| DL-Malic acid | | | 1 | |
| Hypromellose | | | | 1 |
| Sodium hydroxide | | q.s. | q.s. | q.s. |
| Dilute hydrochloric acid | | q.s. | q.s. | q.s. |
| Purified water | | q.s. | q.s. | q.s. |
| pH | | 6 | 6 | 6 |
| Residual rate of AFDX0250 (%) | Light exposure | 96.1 | 94.0 | 82.8 |
| | Shading | 100.5 | 100.8 | 99.9 |

**[Table 9]**

| %(w/v) | | Ex.13 | Ex.14 | Ex.15 | Ex.16 | Ex.17 |
|---|---|---|---|---|---|---|
| AFDX0250 | | 0.001 | 0.001 | 0.001 | 0.001 | 0.001 |
| Sodium citrate hydrate | | 0.2 | 0.2 | 0.2 | 0.2 | 0.2 |
| Trometamol | | 1 | 1 | 1 | 1 | 1 |
| Sodium chloride | | 0.75 | | | | |
| Glycerin | | | 2 | | | |
| D-Mannitol | | | | 4 | | |
| Propylene glycol | | | | | 2 | |
| Hypromellose | | | | | | 1 |
| Sodium hydroxide | | q.s. | q.s. | q.s. | q.s. | q.s. |
| Dilute hydrochloric acid | | q.s. | q.s. | q.s. | q.s. | q.s. |
| Purified water | | q.s. | q.s. | q.s. | q.s. | q.s. |
| pH | | 6 | 6 | 6 | 6 | 6 |
| Residual rate of AFDX0250 (%) | Light exposure | 99.3 | 98.1 | 99.4 | 98.6 | 92.7 |
| | Shading | 101.3 | 100.3 | 100.8 | 99.7 | 99.5 |

**[Table 10]**

| % (w/v) | | Comparative Ex.2 | Comparative Ex.3 | Comparative Ex.4 | Comparative Ex.5 |
|---|---|---|---|---|---|
| AFDX0250 | | 0.001 | 0.001 | 0.001 | 0.001 |
| Sodium citrate hydrate | | 0.2 | 0.2 | 0.2 | 0.2 |
| Sodium dihydrogen phosphate hydrate | | 3 | | | |
| Boric acid | | | 1 | | |
| L-Ascorbic acid | | | | 1 | |
| Ethyl pyruvate | | | | | 1 |
| Sodium hydroxide | | q.s. | q.s. | q.s. | q.s. |
| Dilute hydrochloric acid | | q.s. | q.s. | q.s. | q.s. |
| Purified water | | q.s. | q.s. | q.s. | q.s. |
| pH | | 6 | 6 | 6 | 6 |
| Residual rate of AFDX0250 (%) | Light exposure | 65.8 | 71.3 | 67.8 | 6.2 |
| | Shading | 99.7 | 100.2 | 76.8 | 97.7 |

As clearly shown in Tables 7 to 10, the formulations of Examples 5 to 17 had higher residual ratios of AFDX0250 under the light irradiation as compared to the formulations of Comparative Examples 2 to 5. In particular, the formulations of Examples 7, 8, 10, 11 and 13 to 17 comprising sodium citrate hydrate and trometamol, ε-aminocaproic acid, L-glutamine or DL-malic acid had higher residual ratio of AFDX0250. On the other hand, the formulation of Comparative Example 4 comprising sodium citrate hydrate and L-ascorbic acid had lower residual ratio of AFDX0250 even under light shading. Hence, it was confirmed that the pharmaceutical composition of the present invention had superior stability to light.

### 3. Stability Evaluation Test (3)

The effect of light irradiation on the stability of the pharmaceutical composition comprising the compound of the present invention was evaluated.

### 3-1. Preparation of Test Formulation

Sodium citrate hydrate (2.5 g), citric acid hydrate (0.2 g) and sodium chloride (7.8 g) were dissolved in purified water so as to be exactly 100 mL to prepare 2.5% sodium citrate hydrate/0.2% citric acid hydrate/7.8% sodium chloride solution. AFDX0250 (0.01 g) was dissolved in purified water so as to be exactly 100 mL to prepare 0.01% AFDX0250 solution. Trometamol (5 g) was dissolved in purified water so as to be exactly 100 mL to prepare 5% trometamol solution. The 5% trometamol solution was diluted to prepare 0.5% and 0.05% trometamol solutions.

The 2.5% sodium citrate hydrate/0.2% citric acid hydrate/7.8% sodium chloride solution (10 mL), the 0.01% AFDX0250 solution (10 mL) and purified water were mixed to prepare a solution, sodium hydroxide or dilute hydrochloric acid was added thereto to adjust the pH to 6, and then purified water was added thereto so as to be exactly 100 mL to prepare the formulation of Example 18.

The formulations of Examples 19 to 23 were prepared in a similar process to the preparation process of Example 18 except that the 0.05%, 0.5% or 5% trometamol solution (10 mL or 20 mL) was added to the composition of Example 18. The amount of purified water to be added was suitably changed depending on the amounts of additives.

### 3-2. Test Method

Each of the test formulations prepared in the above 3-1 was filled in a container and exposed to 1.2 million lux·hr of light at 1000 lux/hr or shaded with aluminum foil. The amount of AFDX0250 when exposed to light or shaded was quantified by high performance liquid chromatography to calculate the residual ratio of AFDX0250 (%). The residual ratio of AFDX0250 (%) represents the ratio of AFDX0250 after light exposure or shading when the amount of AFDX0250 in the test formulation is 1000 (%).

### 3-3. Test Results and Discussion

The test results are shown in Table 11.

**[Table 11]**

| %(w/v) | | Ex.18 | Ex.19 | Ex.20 | Ex.21 | Ex. 22 | Ex.23 |
|---|---|---|---|---|---|---|---|
| AFDX0250 | | 0.001 | 0.001 | 0.001 | 0.001 | 0.001 | 0.001 |
| Sodium citrate hydrate | | 0.25 | 0.25 | 0.25 | 0.25 | 0.25 | 0.25 |
| Citric acid hydrate | | 0.02 | 0.02 | 0.02 | 0.02 | 0.02 | 0.02 |
| Trometamol | | | 0.01 | 0.05 | 0.1 | 0.5 | 1 |
| Sodium chloride | | 0.78 | 0.78 | 0.78 | 0.78 | 0.78 | 0.78 |
| Sodium hydroxide | | q.s. | q.s. | q.s. | q.s. | q.s. | q.s. |
| Dilute hydrochloric acid | | q.s. | q.s. | q.s. | q.s. | q.s. | q.s. |
| Purified water | | q.s. | q.s. | q.s. | q.s. | q.s. | q.s. |
| pH | | 6 | 6 | 6 | 6 | 6 | 6 |
| Residual rate of AFDX0250 (%) | Light exposure | 85.3 | 87.8 | 91.6 | 91.4 | 95.2 | 96.1 |
| | Shading | 96.9 | 98.9 | 97.7 | 98.4 | 98.7 | 98.2 |

As clearly shown in Table 11, the formulations of Examples 18 to 23 had high residual ratio of AFDX0250 under the light irradiation. Hence, it was confirmed that the pharmaceutical composition of the present invention had superior stability to light.

### 4. Stability Evaluation Test (4)

The effect of temperature on the stability of the pharmaceutical composition comprising the compound of the present invention was evaluated.

### 4-1. Preparation of Test Formulation

The formulations of Examples 24 to 27 were prepared in a similar process to the preparation process of Example 18. The amounts of AFDX0250 and each additive to be added are as shown in Table 12, and the amount of purified water to be added was suitably changed depending on the amounts of additives.

### 4-2. Test Method

Each of the test formulations prepared in the above 4-1 was stored at each temperature of -20°C and 5°C for 3 months. The amount of AFDX0250 in the test formulation after 3 month storage at each temperature was quantified by high performance liquid chromatography to calculate the residual ratio of AFDX0250 (%). The residual ratio of AFDX0250 (%) represents the ratio of AFDX0250 after storage when the amount of AFDX0250 in the test formulation prior to storage is 100% (%).

### 4-3. Test Results and Discussion

The test results are shown in Table 12.

**[Table 12]**

| %(w/v) | | Ex.24 | Ex.25 | Ex.26 | Ex.27 |
|---|---|---|---|---|---|
| AFDX0250 | | 0.02 | 0.1 | 0.5 | 3 |
| Sodium citrate hydrate | | 0.25 | 0.25 | 0.25 | 0.25 |
| Citric acid hydrate | | 0.02 | 0.02 | 0.02 | 0.02 |
| Sodium chloride | | 0.78 | 0.78 | 0.7 | 0.32 |
| Sodium hydroxide | | q.s. | q.s. | q.s. | q.s. |
| Dilute hydrochloric acid | | q.s. | q.s. | q.s. | q.s. |
| Purified water | | q.s. | q.s. | q.s. | q. s. |
| pH | | 6 | 6 | 6 | 6 |
| Residual rate of AFDX0250 (%) | -20°C/3 months | 100.6 | 100.7 | 100.9 | 101.1 |
| | 5°C/3 months | 100.6 | 100.4 | 100.4 | 100.7 |

As clearly shown in Table 12, the formulations of Examples 24 to 27 had high residual ratios of AFDX0250 in a low temperature of -20°C and 5°C. Hence, it was confirmed that AFDX0250 had superior stability in the pharmaceutical composition of the present invention when the pharmaceutical composition was stored at low temperature.

### 5. Stability Evaluation Test (5)

The effect of temperature on the stability of the pharmaceutical composition comprising the compound of the present invention was evaluated.

### 5-1. Preparation of Test Formulation

The formulation of Examples 28 to 31 were prepared in a similar process to the preparation process of Example 5. The amounts of AFDX0250 and each additive to be added are as shown in Table 13, and the amount of purified water to be added was suitably changed depending on the amounts of additives. The formulations of Examples 28, 29 and 31 were adjusted to pH 5, 5.5 and 6.5, respectively.

### 5-2. Test Method

Each of the test formulations prepared in the above 5-1 was stored under each condition of 5°C and 25°C/40%RH for 6 months. The content of AFDX0250 in the test preparation after 6 month storage was quantified by high performance liquid chromatography to calculate the residual ratio of AFDX0250 (%). The residual ratio of AFDX0250 (%) represents the ratio of AFDX0250 after storage when the amount of AFDX0250 in the test formulation prior to storage is 100% (%).

### 5-3. Test Results and Discussion

The test results are shown in Table 13.

**[Table 13]**

| %(w/v) | | Ex.28 | Ex.29 | Ex.30 | Ex.31 |
|---|---|---|---|---|---|
| AFDX0250 | | 0.01 | 0.01 | 0.01 | 0.01 |
| Sodium citrate hydrate | | 0.5 | 0.5 | 0.5 | 0.5 |
| Sodium hydroxide | | q.s. | q.s. | q.s. | q.s. |
| Dilute hydrochloric acid | | q.s. | q.s. | q.s. | q.s. |
| Purified water | | q.s. | q.s. | q.s. | q.s. |
| pH | | 5 | 5.5 | 6 | 6.5 |
| Residual rate of AFDX0250 (%) | 5°C/6 months | 103.8 | 104.3 | 103.9 | 105.1 |
| | 25°C/40%RH/6 months | 99.7 | 101.7 | 101.5 | 101.8 |

As clearly shown in Table 13, the formulations of Examples 28 to 31 had high residual ratios of AFDX0250 at 5°C and 25°C. Hence, it was confirmed that AFDX0250 had superior stability in the pharmaceutical composition of the present invention with a pH of 6.5 or less when the pharmaceutical composition was stored near room temperature.

### 6. Stability Evaluation Test (6)

The stability of the pharmaceutical composition comprising the compound of the present invention under the storage at high temperature was evaluated.

### 6-1. Preparation of test formulation

The formulations of Examples 32 to 35 and Comparative Example 6 were prepared in a similar process to the preparation process of Example 5. The amounts of AFDX0250 and each additive to be added are as shown in Table 14, and the amount of purified water to be added was suitably changed depending on the amounts of additives. The formulations of Examples 32, 33, 35 and Comparative Example 6 were adjusted to pH 5, 5.5, 6.5 and 7, respectively.

### 6-2. Test Method

Each test formulation prepared in the above 6-1 was stored at 50°C and sampled after 1, 2 and 4 weeks. The amounts of AFDX0250 in the test preparations at each time point was quantified by high performance liquid chromatography to calculate the residual ratio of AFDX0250 (%). The residual ratio of AFDX0250 (%) represents the ratio of AFDX0250 after storage when the amount of AFDX0250 in the test formulation prior to storage is 100% (%).

### 6-3. Test Results and Discussion

The test results are shown in Table 14.

**[Table 14]**

| % (w/v) | | Ex.32 | Ex.33 | Ex.34 | Ex.35 | Comparative Ex.6 |
|---|---|---|---|---|---|---|
| AFDX0250 | | 0.01 | 0.01 | 0.01 | 0.01 | 0.01 |
| Sodium citrate hydrate | | 0.5 | 0.5 | 0.5 | 0.5 | 0.5 |
| Sodium hydroxide | | q.s. | q.s. | q.s. | q.s. | q.s. |
| Dilute hydrochloric acid | | q.s. | q.s. | q.s. | q.s. | q.s. |
| Purified water | | q.s. | q.s. | q.s. | q.s. | q.s. |
| pH | | 5 | 5.5 | 6 | 6.5 | 7 |
| Residual rate of AFDX0250 (%) | 50°C/1 week | 98.0 | 96.7 | 96.8 | 94.0 | 90.6 |
| | 50°C/2 weeks | 93.9 | 94.8 | 94.2 | 90.2 | 80.1 |
| | 50°C/4 weeks | 87.4 | 88.9 | 87.1 | 81.3 | 64.7 |

As clearly shown in Table 14, the formulations of Examples 32 to 35 had higher residual ratios of AFDX0250 in a high temperature of 50°C as compared to that of Comparative Example 6. Hence, it was confirmed that when the pharmaceutical composition of the present invention with a pH of 6.5 or less was stored at high temperature, AFDX0250 had superior stability in the pharmaceutical composition.

### INDUSTRIAL APPLICABILITY

The pharmaceutical composition comprising (+)-11-[2-[2-[(diethylamino)methyl]-1-piperidinyl]acetyl]-5,11-dihydro-6H-pyrido[2,3-b][1,4]benzodiazepin-6-one or a salt thereof of the present invention is stable, and thus can be used stably as a medicament over a long period.

## Claims

1. A pharmaceutical composition with a pH of 6.5 or less comprising (+)-11-[2-[2-[(diethylamino)methyl]-1-piperidinyl]acetyl]-5,11-dihydro-6H-pyrido[2,3-b][1,4]benzodiazepin-6-one or a salt thereof.

2. The pharmaceutical composition according to claim 1, wherein the pH is 4 to 6.5.

3. A pharmaceutical composition comprising (+)-11-[2-[2-[(diethylamino)methyl]-1-piperidinyl]acetyl]-5,11-dihydro-6H-pyrido[2,3-b][1,4]benzodiazepin-6-one or a salt thereof and one or more buffering agents selected from the group consisting of citric acid, acetic acid, malic acid, an amine, an amino acid and a salt thereof.

4. The pharmaceutical composition according to claim 1 or 2, which comprises one or more buffering agents selected from the group consisting of citric acid, acetic acid, malic acid, an amine, an amino acid and a salt thereof.

5. The pharmaceutical composition according to claim 3 or 4 comprising citric acid or a salt thereof, wherein the pharmaceutical composition further comprises any of acetic acid or a salt thereof, malic acid or a salt thereof, an amine or a salt thereof, or an amino acid or a salt thereof as a buffering agent.

6. The pharmaceutical composition according to any one of claims 3 to 5, wherein the amine is trometamol.

7. The pharmaceutical composition according to any one of claims 3 to 6, wherein the amino acid is ε-aminocaproic acid, glycine or L-glutamine.

8. The pharmaceutical composition according to any one of claims 3 to 5, wherein the buffering agent is citric acid or a salt thereof and trometamol or a salt thereof.

9. The pharmaceutical composition according to any one of claims 1 to 8, wherein the content of (+)-11-[2-[2-[(diethylamino)methyl]-1-piperidinyl]acetyl]-5,11-dihydro-6H-pyrido[2,3-b][1,4]benzodiazepin-6-one or a salt thereof in the pharmaceutical composition is 0.0001 to 5% (w/v).

10. The pharmaceutical composition according to any one of claims 3 to 9, wherein the content of the buffering agent in the pharmaceutical composition is 0.001 to 10% (w/v).

11. The pharmaceutical composition according to any one of claims 3 to 10, wherein the content of the buffering agent in the pharmaceutical composition is 0.1 to 20000 parts by weight or 0.01 to 1000 parts by weight per part by weight of (+)-11-[2-[2-[(diethylamino)methyl]-1-piperidinyl]acetyl]-5,11-dihydro-6H-pyrido[2,3-b][1,4]benzodiazepin-6-one or a salt thereof.

12. The pharmaceutical composition according to any one of claims 1 to 11 for ocular topical administration

13. The pharmaceutical composition according to claim 12, wherein the ocular topical administration is instillation administration, conjunctival sac administration, intravitreal administration, sub-conjunctival administration or sub-Tenon's administration.

14. The pharmaceutical composition according to any one of claims 1 to 13 which is an eye drop, an eye gel or an injection.

15. The pharmaceutical composition according to any one of claims 1, 2 and 4 to 14 stored at 50°C or less.

16. A method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof in a pharmaceutical composition comprising the same, which comprises adjusting the pH of the pharmaceutical composition to 6.5 or less.

17. A method of stabilizing (+)-11-[2-[2-[(diethylamino)methyl]-1-piperidinyl]acetyl]-5,11-dihydro-6H-pyrido[2,3-b][1,4]benzodiazepin-6-one or a salt thereof in a pharmaceutical composition comprising the same, which comprises adding one or more buffering agents selected from the group consisting of citric acid, acetic acid, malic acid, an amine amino acid or a salt thereof into the pharmaceutical composition.
